# EUROPEAN PATENT APPLICATION

(11) **EP 1 642 903 A1**
(43) Date of publication of application: **05.04.2006**
(21) Application number: 04746360.9
(22) Date of filing: 24.06.2004
(51) Int. Cl.: C07K 7/06, C07K 7/08, C12N 15/09, A61K 38/03, A61K 38/04, A61P 9/10, A61P 25/28, A61P 3/10, A61P 37/06

(54) **PEPTIDE HAVING APOPTOSIS-INHIBITING ACTIVITY**

(30) Priority: 24.06.2003 JP 2003180131
(71) Applicant: Keio University, Tokyo 108-0073 (JP)
(72) Inventor: IMOTO, Masaya, c/o Keio Univ. Faculty of Science, Yokohama-shi, Kanagawa 223-0061 (JP); TABE, Hirokazu, c/o Keio Univ. Faculty of Science, Yokohama-shi, Kanagawa 223-0061 (JP); TASHIRO, Etsu, c/o Keio Univ. Faculty of Science, Yokohama-shi, Kanagawa 223-0061 (JP); OHMORI, Yusuke, c/o Keio Univ. Faculty of Science, Yokohama-shi, Kanagawa 223-0061 (JP)
(74) Representative: Horner, Martin Grenville
(86) International application number: PCT/JP2004/008891
(87) International publication number: WO 2004/113367

(57) **Abstract**

A peptide consisting of part of the amino acid sequence of SEQ ID No: 8 and having an amino acid sequence of SEQ ID No: 1 or SEQ ID No: 2, e.g., a peptide consisting of the amino acid sequence of any one of SEQ ID Nos: 1 to 5, has an excellent apoptosis-suppressive activity. Therefore, a peptide consisting of part of the amino acid sequence of SEQ ID No: 8 and having an amino acid sequence of SEQ ID No: 2, particularly SEQ ID No: 1, is useful as a pharmaceutical composition for a Bax-dependent apoptosis-induced disease, such as a neurodegenerative disease.

## Description

### TECHNICAL FIELD

The present invention relates to peptides with apoptosis-suppressive activity, polynucleotides, recombinant vectors, cells or viruses, fusion polynucleotides, fusion proteins, pharmaceutical compositions for Bax-dependent apoptosis-induced diseases, suppressors that suppress the translocation of Bax to mitochondria, methods for suppressing apoptosis-promoting activity, and methods for treating, preventing, or suppressing progression of, Bax-dependent apoptosis-induced diseases.

### BACKGROUND ART

Neurodegenerative diseases, such as brain ischemia and Alzheimer's disease, are known to be diseases resulting from neuronal death (apoptosis). This apoptosis occurs as a result of induction of activation of apoptosis-promoting proteins, such as Bax.
For this reason, there has been a demand for the development of apoptosis-suppressing substances as therapeutic agents for neurodegenerative diseases or suppressors of their progression.

In recent years, it has been reported that a polypeptide containing a Bax ART (apoptotic regulation of targeting) domain (19 amino acids at the N-terminus of GenBank accession number AAA03619 or AAA03620) is useful for suppression of apoptosis-promoting activity (refer to, e.g., National Publication of International Patent Application No. 2002-539760).

### DISCLOSURE OF THE INVENTION

### PROBLEMS TO BE SOLVED BY THE INVENTION

The object of the present invention is to provide peptides with apoptosis-suppressive activity, polynucleotides, recombinant vectors, cells or viruses, fusion polynucleotides, fusion proteins, or suppressors that suppress the mitochondrial translocation of Bax, all of which are useful for apoptosis-induced diseases and for the treatment, prevention, or suppression of progression, of such diseases, together with suppression of apoptosis-promoting activity.

### MEANS FOR SOLVING THE PROBLEM

The present inventors have assiduously studied to provide a pharmaceutical composition useful for diseases caused by apoptosis and, as a result, found that the peptide (SEQ ID NO: 2) consisting of the amino acid sequence from position 13 to position 20 of the peptide MDGSGEQPRGGGPTSSEQIM (the peptide consisting of the N-terminal 20-amino acid sequences of Bax: SEQ ID NO: 8) consisting of the Bax ART domain has an strong apoptosis-suppressive activity. Further, the inventors have found that the peptide (SEQ ID NO: 1) consisting of the amino acid sequence from position 14 to position 20 of the ART domain has a stronger apoptosis- suppressive activity than the peptide (SEQ ID NO: 2) consisting of the amino acid sequence from position 13 to position 20 of the ART domain. Accordingly, it is suggested that the peptide consisting of the amino acid sequence shown in SEQ ID NO: 2, particularly the peptide consisting of the amino acid sequence shown in SEQ ID NO: 1, is the core domain of the apoptosis-suppressive activity inART. It is therefore inferred that peptides including the partial sequence of ART consisting of the amino acid sequence shown in SEQ ID NO: 8 and the amino acid sequence shown in SEQ ID NO: 1 or SEQ ID NO: 2 have excellent apoptosis-suppressive activity. As a matter of fact, as will be shown in Examples, a partial sequence of ART including the amino acid sequence shown in SEQ ID NOs : 1 to 5 has an excellent apoptosis-suppressive activity. The inventors have thus accomplished the present invention.

That is, the peptide according to the present invention consists of a part of an amino acid sequence of SEQ ID No: 8 and has the amino acid sequence of SEQ ID No: 1.

Alternatively, the peptide according to the present invention consists of a part of an amino acid sequence of SEQ ID No: 8 and has the amino acid sequence of SEQ ID No: 2.

An example of such a peptide is a peptide consisting of an amino acid sequence shown in any of SEQ ID NOs: 1-5. Alternatively, the aforementioned peptide may be a peptide consisting of an amino acid sequence in which one or a few nucleotides are deleted, substituted, or added, and having apoptosis-suppressive activity. Examples of such a peptide include PTAAEQIM (SEQ ID NO: 6), VTSSEQIM (SEQ ID NO: 7), etc. It should be noted that the peptide according to the present invention does not include ART.

The polynucleotide or the polynucleotide having the nucleotide sequence complementary to its nucleotide sequence according to the present invention encodes a peptide with apoptosis-suppressive activity. These polynucleotides encode peptides more useful than the peptide consisting of a Bax ART domain, as will be described later. These polynucleotides may form double-stranded DNA or single-stranded DNA, or may form double-stranded RNA or single-stranded RNA.

The recombinant vector according to the present invention contains the aforementioned polynucleotide. Examples of the recombinant vector containing the aforementioned polynucleotide include viral vectors, such as an adenovirus vector, a retroviral vector, a lentivirus vector, an adeno-associated virus vector, a Herpesvirus vector, and HIV vector, as well as transposons or plasmid vectors. The cell according to the present invention contains the aforementioned recombinant vector. The host cell used as the cell according to the present invention may be any cell ranging from microbial cell, such as Escherichia coli, to a mammalian cell. The virus according to the present invention is not limited to any virus as long as it can be used for gene transfer. Examples of such viruses include adenoviruses, retroviruses, lentiviruses, adeno-associated viruses, and herpesviruses, HIV, etc.

Further, the polynucleotide according to the present invention is a fusion polynucleotide of the aforementioned polynucleotide and a polynucleotide encoding a tag and is constructed such that the peptide encoded by these polynucleotides can be expressed as a fused peptide. The tag is not limited to any specific one as long as it is a molecule that can be used to facilitate purification of the peptide with apoptosis-suppressive activity expressed in cells. Examples of such tags include His tag, GST tag, FLAG tag, etc.

The fusion protein according to the present invention is formed by fusing the peptide according to the present invention to a cell-permeable peptide for delivering the aforementioned peptide into cells. Examples of cell-permeable peptides include hydrophobic signal peptides (J. Biol. Chem., 270, 14255- 14258, 1995, Nat. Biotechnol., 16, 370- 375, 1998), HIV-1 TAT peptides, etc. In addition, antimicrobial peptides, such as magainin 2, can be used as cell-permeable peptides.

The pharmaceutical composition for Bax-dependent apoptosis-induced diseases according to the present invention contains as an active ingredient at least one substance selected from the group consisting of the aforementioned peptide, polynucleotide, recombinant vector, cell or virus, and fusion protein.

In the method for treating, preventing, or suppressing progression of Bax-dependent apoptosis-induced diseases according to the present invention, at least one substance selected from the group consisting of the aforementioned peptide, polynucleotide, recombinant vector, cell or virus, and fusion protein is used. It should be noted that vertebrates afflicted with a Bax-dependent apoptosis-induced diseases may be humans or vertebrates other than humans, such as mice and rats.

Bax-dependent apoptosis-induced diseases are diseases caused by apoptosis induced by Bax. Examples include ischemic diseases, neurodegenerative diseases, diabetes, autoimmune diseases, allergic diseases, etc. Examples of ischemic diseases include brain ischemia, ischemia retinae, ischemic heart disease, etc. Examples of neurodegenerative diseases include Alzheimer's disease, Parkinson's disease, poly glutamine disease, prion disease, amyotrophic lateralsclerosis (ALS), acquired immunodeficiency syndrome (AIDS) encephalopathy, etc. Examples of autoimmune diseases include multiple sclerosis, myasthenia gravis, etc.

The suppressor that suppresses the mitochondrial translocation of Bax according to the present invention contains as an active ingredient at least one substance selected from the group consisting of the aforementioned peptide, polynucleotide, recombinant vector, cell or virus, and fusion protein.

A method for suppressing apoptosis-promoting activity according to the present invention uses at least one substance selected from the group consisting of the aforementioned peptide, polynucleotide, recombinant vector, cell or virus, and fusion protein.

### CROSS REFERENCE TO RELATED APPLICATIONS

This application claims the benefit of priority to Japanese Patent Application No. 2003-180131, filed on June 24, 2003, which is incorporated herein by reference.

### BRIEF DESCRIPTION OF THE DRAWINGS

FIG. 1 shows the effects of the wild type ART and the mutated ARTs on Bax-induced apoptosis-promoting activity in Example 1 according to the present invention.
FIG. 2 shows the effects of the amounts of the wild type ART and mutated ART expression vectors on Bax-induced apoptosis-promoting activity in Example 2 according to the present invention.
FIG. 3 shows the expression levels of the wild type ART and the mutated ART examined by Western blotting and the effects of the wild type ART and the mutated ART on Bax-induced apoptosis-promoting activity at each expression level in Example 2 according to the present invention.
FIG. 4 shows the amino acid sequence of HTAI320 prepared in Example 3 according to the present invention.
FIG. 5 shows the effect of HTAI320 on Bax-induced apoptosis-promoting activity in Example 4 according to the present invention.
FIG. 6 shows the effect of TAT-ART1320 on Bax or Bak-induced apoptosis in Example 5 according to the present invention.
FIG. 7 shows the suppressive effect of TAT-ARTI320 on STS-induced Bax-dependent apoptosis in Example 6 according to the present invention.
FIG. 8 shows the effect of TAT-ARTI320 on STS-induced mitochondrial translocation of Bax in Example 7 according to the present invention.
FIG. 9 shows the effect of TAT-ART1320, TAT-ART1420, or TAT-ART1320P13V on STS-induced apoptosis in Example 8 according to the present invention.
FIG. 10 shows the suppressive effect of TAT-ART1320 on cell death of hippocampal neurons isolated from brain ischemia model gerbils in Example 9 according to the present invention.

### BEST MODE FOR CARRYING OUT THE INVENTION

It has been hypothesized that the Bax ART domain suppresses apoptosis-promoting activity of Bax by suppressing mitochondrial localization of Bax through intramolecular binding to the transmembrane domain. As a matter of fact, Bax lacking the ART domain has a higher apoptosis-promoting activity than the wild type Bax. Apoptosis-promoting activity of Bax can also be suppressed by expressing the peptide (hereinafter abbreviated as ART) that has only the ART domain amino acid sequences (hereinafter abbreviated as ART sequences).

The present inventors confirmed that Bax-induced cell death is suppressed by expressing a construct (GFP-ART) in cells through gene transfer, which is prepared by fusing ART-encoding nucleotide sequence (SEQ ID NO: 9) to the GFP gene. However, examination of the binding of GFP-ART to Bax led to experimental results contradictory to the previously-held hypothesis: it was found that ART expressed independently inhibited apoptosis-promoting activity induced by Bax without binding directly to Bax.

Thus, the present inventors constructed a wide variety of deletion mutants and point mutants of ART and investigated Bax-induced inhibition of apoptosis-promoting activity. As a result, it was found that a construct containing polynucleotides that encode the peptide consisting of part of ART, for example the peptide consisting of a sequence from proline in the 13th position through methionine in the 20th position from the N-terminus of the ART sequence exhibits a 10-to 100-fold stronger apoptosis-suppressive activity than ART.

Further, the inventors found that the peptide having the amino acid sequence shown in SEQ ID NO: 1 or SEQ ID NO: 7 has a stronger suppressive activity on staurosporine (STS)-induced apoptosis than the peptide having the amino acid sequence shown in SEQ ID NO: 2.

Embodiments of the present invention accomplished based on the above-described findings are hereinafter described in detail by giving Examples. Unless otherwise explained, methods described in standard sets of protocols such as J. Sambrook and E. F. Fritsch & T. Maniatis (Ed.),"Molecular Cloning, a Laboratory Manual (3rd edition), Cold Spring HarborPress and Cold Spring Harbor, New York (1989); and F. M. Ausubel, R. Brent, R. E. Kingston, D. D. Moore, J. G. Seidman, J. A. Smith, and K. Struhl (Ed.), "Current Protocols in Molecular Biology," John Wiley & Sons Ltd., or alternatively, their modified/changed methods are used. When using commercial reagent kits and measuring apparatus, unless otherwise explained, protocols attached to them are used. The obj ect, characteristics, and advantages of the present invention as well as the idea thereof will be apparent to those skilled in the art from the descriptions given herein. It is to be understood that the embodiments and specific examples of the invention described herein below are to be taken as preferred examples of the present invention. These descriptions are only for illustrative and explanatory purposes and are not intended to limit the invention to these embodiments or examples. It is further apparent to those skilled in the art that various changes and modifications may be made based on the descriptions given herein within the intent and scope of the present invention disclosed herein.

### (1) A peptide with apoptosis-suppressive activity

The peptide with apoptosis-suppressive activity according to the present invention consists of a part of the ART sequence and has the amino acid sequence shown in SEQ ID NO: 2 (particularly, the amino acid sequence shown in SEQ ID NO: 1). This peptide has a stronger apoptosis-suppressive activity than that possessed by ART, especially the activity induced by Bax. Specific examples of the peptide include the peptide consisting of the amino acid sequence (SEQ ID NO: 1) from threonine in the 14th position to methionine in the 20th position from the N-terminus of the ART sequence; the peptide consisting of the amino acid sequence (SEQ ID NO: 2) from proline in the 13th position to methionine in the 20th position from the N-terminus of the ART sequence; the peptide consisting of the amino acid sequence (SEQ ID NO: 3) from glycine in the 10th position to methionine in the 20th position from the N-terminus of the ART sequence; the peptide consisting of the amino acid sequence (SEQ ID NO: 4) from glutamine in the 7th position to a methionine in the 20th position from the N-terminus of the ART sequence; the peptide consisting of the amino acid sequence (SEQ ID NO: 5) from serine in the 4th position to methionine in the 20th position from the N-terminus of the ART sequence, etc. In addition, the peptide (SEQ ID NO: 6) in which serines at positions 3 and 4 are substituted with alanines in the amino acid sequences shown in SEQ ID NO: 2; and the peptide (SEQ ID NO: 7) in which proline at position 1 is substituted with valine in the amino acid sequences shown in SEQ ID NO: 2 have a similar apoptosis-suppressive activity to that of the peptide consisting of the amino acid sequence shown in SEQ ID NO: 2. Therefore, the peptide with apoptosis-suppressive activity, the fusion protein of the aforementioned peptide and an intracellularly-delivered peptide, the polynucleotide encoding the aforementioned peptide or the aforementioned fusion protein, the recombinant vector containing the aforementioned polynucleotide, the cell or virus containing the aforementioned recombinant vector, etc. according to the present invention can provide a peptide with apoptosis-suppressive activity and are accordingly useful as pharmaceutical compositions for apoptosis-induced diseases.

Further, the peptide consisting of the amino acid sequence shown in SEQ ID NO: 2 has the effect of suppressing SIS-induced mitochondrial translocation of Bax. Therefore, the peptide with apoptosis-suppressive activity according to the present invention, the fusion protein of the aforementioned peptide and an intracellularly-delivered peptide, the polynucleotide encoding the aforementioned peptide or the aforementioned fusion protein, the recombinant vector containing the aforementioned polynucleotide, the cell or the virus containing the aforementioned recombinant vector, etc. can also suppress mitochondrial translocation of Bax and are accordingly useful as suppressors that suppress the mitochondrial translocation of Bax.

It should be noted that the peptide with apoptosis-suppressive activity according to the present invention can be more efficiently prepared because it consists of an amino acid sequence shorter than ART.

### (2) Method for preparing pharmaceutical compositions that have apoptosis-suppressive activity

Peptides with apoptosis-suppressive activity may be organic-chemically synthesized, or may be obtained by hydrolyzing proteins using various methods such as an enzymatic method. Alternatively, recombinant vectors may be prepared by inserting a polynucleotide encoding a peptide with apoptosis-suppressive activity into an expression vector harboring a suitable enhancer/promoter. It is more preferable to express a fusion protein by inserting a fusion polynucleotide in which a polynucleotide encoding a tag (e.g., His tag, GST tag, or the like) is fused to a polynucleotide encoding a peptide with apoptosis-suppressive activity; and then introducing the recombinant vector containing this fusion polynucleotide into bacteria (e.g., Escherichia coli, salmonella, etc.), yeast, animal cells, etc. to express the fusion protein, because the fusion protein that has been expressed can be purified using a tag. In addition, mRNA obtained by transcribing the aforementioned polynucleotide or the aforementioned fusion polynucleotide in vitro may be translated in an in vitro translation system using rabbit reticulocyte lysate, Escherichia-coli S30 extract, malt extract, wheat germ extract, etc. The fusion protein can be purified similarly using a tag. It should be noted that a tag can be removed at the final step to purify only a peptide with apoptosis-suppressive activity by HPLC (high-performance (speed) liquid chromatography) or the like. The peptide with apoptosis-suppressive activity thus prepared according to the present invention can be used to treat, prevent, or suppress the progression of, apoptosis-induced diseases.

Here, a recombinant vector capable of expressing a peptide with apoptosis-suppressive activity may be used as a pharmaceutical composition in place of a peptide with apoptosis-suppressive activity. For example, by introducing the aforementioned vector into the targeted diseased area, it becomes possible to express a peptide with apoptosis-suppressive activity.

Alternatively, a cell or a virus containing the aforementioned recombinant vector may be used as a pharmaceutical composition. Such a cell or virus can release a peptide with apoptosis-suppressive activity, which is expressed in a recombination cell in the target site in the living body. The recombinant cells according to the present invention can be prepared by introducing into, for example, animal cells a recombinant vector capable of expressing a peptide with apoptosis-suppressive activity by the electroporation method, microinjection method, lipofection method, viral infection method using a viral vector (e.g., an adenovirus or a retrovirus, etc.), transfection method with calcium, or the like. Further, cells, bacteria, or viruses harboring a recombinant vector containing DNA encoding the aforementioned peptide may be administered to patients. In this case, to minimize toxicity and prevent side effects, the bacteria and viruses should preferably be attenuated in advance. For example, a retrovirus or an adenovirus engineered to infect, but not to replicate, may contain a recombinant vector capable of expressing a peptide with apoptosis-suppressive activity.

### (3) Administration of pharmaceutical composition

As mentioned above, the peptide with apoptosis-suppressive activity, the fusion protein of the aforementioned peptide and an peptide which can deliver the peptide with apoptosis-suppressive activity into cells, the recombinant vector capable of expressing the aforementioned peptide or aforementioned fusion protein, the cell or virus, etc. containing the aforementioned recombinant vector are useful as pharmaceutical compositions for apoptosis-induced diseases.

In addition, liposomes encapsulating a peptide with apoptosis-suppressive activity or a polynucleotide encoding the peptide are useful as pharmaceutical compositions. In this case, Trans IT In Vivo Gene Delivery System (TAKARA) or the like can be used. In addition, polymeric micelles in which the aforementioned peptide, aforementioned polynucleotide, or the like is encapsulated in polymeric micelles having excellent characteristics as drug carriers are also useful as pharmaceutical compositions.

Alternatively, complexes of a plasmid capable of expressing a peptide with apoptosis-suppressive activity and a polymeric carrier can deliver the plasmid to a target tissue, where the drug can be released. Such complexes are therefore useful as pharmaceutical compositions for apoptosis-induced diseases. Polymeric carriers refer to the substances with which the aforementioned polynucleotide can be introduced into cells. Examples of polymeric carriers include polylysine, polyethyleneimine, porotamine, chitosan, synthetic peptides, dendrimers, or the like.

In addition, the pharmaceutical composition may further contain a suitable pharmacologically acceptable excipient or base depending on the site or purpose of administration.

It is preferable to administer the pharmaceutical composition thus prepared that contains as an active ingredient a peptide with apoptosis-suppressive activity, a fusion protein of the aforementioned peptide and an intracellularly-delivered peptide, a recombinant vector capable of expressing the aforementioned peptide or aforementioned fusion protein, a cell, a virus, or the like containing the aforementioned recombinant vector, and that contains other components for administration as described above directly to the vicinity of the targeted diseased area in a human or a vertebrate other than a human. Alternatively, it may be preferable to administer the pharmaceutical composition parenterally, orally, intradermally, subcutaneously, intravenously, intramuscularly, or intraperitoneally.

Thus, by introducing a peptide with apoptosis-suppressive activity into a living body, it becomes possible to suppress apoptosis that causes disease.

### (4) Apoptosis-induced diseases

The pharmaceutical composition according to the present invention, which suppresses Bax-induced apoptosis, is applicable to any Bax-dependent apoptosis-induced diseases caused by Bax-induced apoptosis.

For example, previously, it was reported that Bax antisense suppresses apoptosis in ischemia retinae, which is a kind of ischemic disease (Neurosci. Res. Suppl. 22, pp. S357, 1998). In addition, it has been known that Bax is involved in cell death associated with brain ischemia (3rd Annual Meeting of the Japanese Society of Mitochondrial Research and Medicine, Book of Abstracts, No. S1) and in neuronal death associated with Alzheimer's disease or its developing process.

Another example is given here: In the Akita mice, a diabetic mouse model, endoplasmic reticulum stress-mediated apoptosis in pancreatic β cells occurs. This apoptosis is reported to be induced through the pathway of endoplasmic reticulum stress, CHOP induction, the mitochondrial translocation of Bax, the activation of mitochondrial pathway, and apoptosis (3rd Annual Meeting of the Japanese Society of Mitochondrial Research and Medicine, Book of Abstracts, No. S1).

In addition, it is known that, in the Bax-knockout mice, the development of myelin oligodendrocyte glycoprotein (MOG)-induced autoimmune encephalomyelitis, which is an animal model of the multiple sclerosis, a type of autoimmune disease, is suppressed (Neurosci Lett. 2004 Apr 15, 359 (3), 139-42), and that the expression level of Bax increases with the increasing level of myasthenia gravis (Eur J Cardiothorac Surg. 2001 Oct, 20(4), 712-21.). Thus, it has been revealed that Bax is also involved in the apoptosis that causes these autoimmune diseases.

Thus, the pharmaceutical composition according to the present invention can be a therapeutic, preventive, or progression-suppressive agent against ischemic diseases, such as myocardial infarction, liver ischemia, brain ischemia, and ischemia retinae; neurodegenerative diseases, such as an Alzheimer's disease, Parkinson's disease, poly glutamine disease, prion disease, amyotrophic lateralsclerosis (ALS), and acquired immunodeficiency syndrome (AIDS); diabetes; autoimmune diseases, such as multiple sclerosis and myasthenia gravis, etc.

Iodinated contrast media is known to cause apoptosis-induced renal damage. Iodinated contrast media also induces apoptosis in incubated renal tubular cells. It has been shown that the expression level of Bax is increased by iodinated contrast media in incubated renal tubular cells (Kidney Int., 64, 2052-2063, 2003). Therefore, by using the pharmaceutical composition according to the present invention when iodinated contrast media are used (in imaging of blood vessels or organs), it is possible to suppress Bax-induced apoptosis and thereby to prevent renal damage.

### EXAMPLES

Examples according to the present invention will be described in detail hereinbelow.

### EXAMPLE 1

### Construction of mutated ART plasmids

### 1-1 Construction of pCI-GFP-ART Plasmid

First, construction of mutated ART plasmids is explained. Sense primer GFP-ART(S) located upstream of the GFP gene and antisense primer GFP-ART(AS) having the nucleotide sequence complementary to the sequence (SEQ ID NO: 9) encoding the BAX ART sequence (SEQ ID NO: 8) on the 5' end and located downstream of the GFP gene were synthesized. PCR reactions were performed with AccuPower PCR PreMix (manufactured by BIONEER), using pEGFP-CI (manufactured by Clontech) as a template, to obtain the insert, GFP-ART, which was then subcloned into pCI-neo Vector (manufactured by Promega) to obtain pCI-GFP-ART (pCI-GFP-ART /1-20) plasmid.

GFP-ART(S):
5′-CGGTATCAAGTTATAGCTAGCGCTACCGGTC-3′ (SEQ ID NO: 10)

GFP-ART(AS):
5'-CCGCTAGCTACATGATCTGCTCAGAGCTGGTGGCCCCCGCCTCTGGG
CTGCTCCCCGGACCCGTCCATCTTGTACAGCTCGTCCATG-3' (SEQ ID NO: 11)

### 1-2 Construction of pCI-GFP-ART/1-9 plasmid and pCI-GFP-ART/1-16 plasmid

PCR reactions were performed, in conjunction with a sense primer upstream of the GFP gene, using pCI-GFP-ART as template and a nucleotide sequence corresponding to positions 1 to 9 or 1 to 16 of the amino acid sequence of the Bax ART domain as a antisense primer to obtain the inserts, GFP-ART 1-9 and GFP-ART/1-16, having the mutated ART sequence of interest. The inserts were subcloned into pCI-neo vector to obtain pCI-GFP-ART/1-9 and pCI-GFP-ART/1-16 plasmids.

### <PCR primers for GFP-ART/1-9>

GFP(S):
5′-CCGTAGCGATAAGGAATTCATGGTGAGCAAGGGCGAGGAG-3′ (SEQ ID NO:
12)

GFP-ART/1-9(AS):
5'-CGGCTATCGGTTACATGAATCCATGGTGAGCAAGGGCGAGGAG-3' (SEQ ID
NO: 13)

### <PCR primers for GFP-ART/1-16>

GFP(S):
5′-CCGTAGCGATAAGGAATTCATGGTGAGCAAGGGCGAGGAG-3′ (SEQ ID NO:
12)

GFP-ART/1-16(AS):
5′-CGGTATTACTAATCTCGCAATTCCTAAGAGCTGGTGGCCCCCCGC-3′ (SEQ ID
NO: 14)

### 1-3 Construction of pCT-GFP-ART/4-20 plasmid, pCT-GFP-ART/7-20 plasmid, pCI-GFP-ART/10-20 plasmid, and GFP-ART/13-20 plasmid

PCR reactions were performed using the sense primer GFP (S) and the antisense primer GFP ART (AS) shown below having the nucleotide sequence complementary to the sequence encoding the mutated ART sequence of interest on the 5' end and located downstream of the GFP gene with AccuPower PCR PreMix (manufactured by BIONEER), using pEGFP-CI (manufactured by Clontech) as template, to obtain the insert encoding the mutated ART fused to GFP. The insert was subcloned into pCI-neo vector (manufactured by Promega) to obtain pCI-GFP-ART/4-20 plasmid, pCI-GFP-ART/7-20 plasmid, pCI-GFP-ART/10-20 plasmid, and pCI-GFP-ART/13-20 plasmid.

### <PCR primers for GFP-ART /4-20>

GFP(S):
5'-CCGTAGCGATAAGGAATTCATGGTGAGCAAGGGCGAGGAG-3' (SEQ ID NO:
12)

GFP-ART/4-20(AS):
5′-CGGAGTTAGATACATATCAAGAATTCCTACATGATCTGCTCAGAGCTGGT
GGGCCCCCCGCCTCTGGGCTGCTCCCCGGACTTGTACAGCTCGTCCATG-3′
(SEQ ID NO: 15)

### <PCR primers for GFP-ART/7-20>

GFP(S):
5′-CCGTAGCGATAAGGAATTCATGGTGAGCAAGGGCGAGGAG-3′ (SEQ ID NO:
12)

GFP-ART/7-20(AS):
5'-CGGACGTAGCGAGATATAGTAGAATTCCTACATGATCTGCTCAGAGCTG
GTGGGCCCCCCGCCTCTGGGCTGCTTGTACAGCTCGTCCATG-3' (SEQ ID NO: 16)

### <PCR primers for GFP-ART/10-20>

GFP(S):
5′-CCGTAGCGATAAGGAATTCATGGTGAGCAAGGGCGAGGAG-3′ (SEQ ID NO:
12)

GFP-ART/10-20(AS):
5'-CCGATTACCGCTTAGAGAATTCCTACATGATCTGCTCAGAGCTGGTGGG
CCCCCCGCCCTTGTACAGCTCGTCCATG-3' (SEQ ID NO: 17)

### <PCR primers for GFP-ART/13-20>

GFP(S):
5'-CCGTAGCGATAAGGAATTCATGGTGAGCAAGGGCGAGGAG-3' (SEQ ID NO:
12)

GFP-ART/13-20(AS):
5′-CCGCGTACGATGGATGTCGAATTCCTACATGATCTGCTCAGAGCTGGTG
GGCTTGTACAGCTCGTCCATG-3′ (SEQ ID NO: 18)

### EXAMPLE 2

### Evaluation of apoptosis-suppressive activity of the mutated ARTs

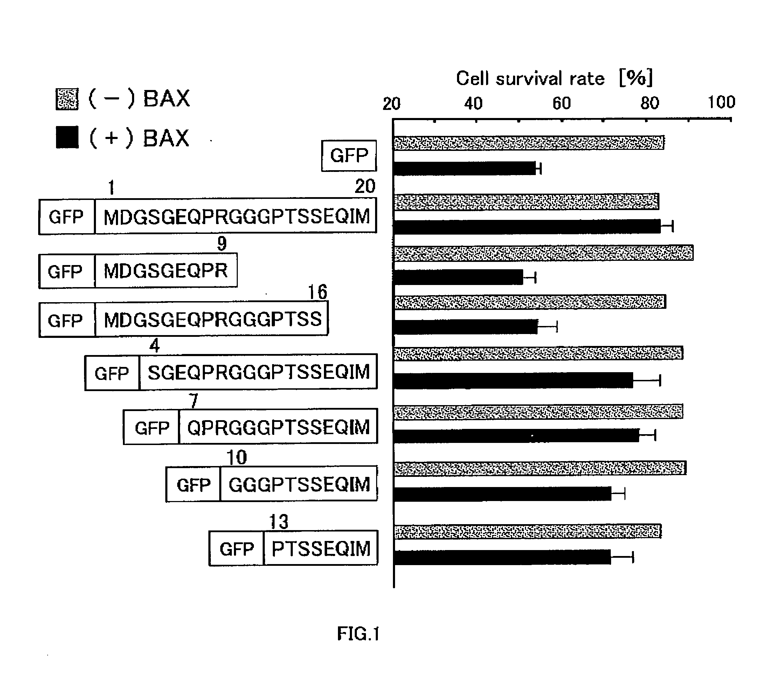

The inhibitory effect of the mutated ARTs on Bax-induced apoptosis-promoting activity were examined using each of the plasmids prepared in Example 1. HEK 293T cells were plated on 12-well plates at 1 x 10⁵ cells/well and incubated overnight. Subsequently, cells were transfected with 0.1 ng/ml wild type or a mutated ART plasmid and pCI-Bax plasmid by lipofection using Opti-MEM (manufactured by GibcoBRL) and Lipofectamine (manufactured by GibcoBRL). After five hours, the medium was changed to DMEM (manufactured by Nissui Pharmaceutical Co., Ltd.) containing 10% FBS (manufactured by Bioserum) and the cell survival rate after 40 hours was evaluated by the trypan blue exclusion method. The results are shown in FIG. 1.
As a result, the survival rate of the cells into which pCI-GFP-ART/4-20 plasmid, pCI-GFP-ART/7-20 plasmid, pCI-GFP-ART/10-20 plasmid, and pCI-GFP-ART/13-20 plasmid had been introduced was not significantly different from that of the cells into which pCI-GFP-ART plasmid had been introduced. Therefore, it was revealed that the C-terminus of the ART sequence is an important region in inhibiting Bax-induced apoptosis-promoting activity.

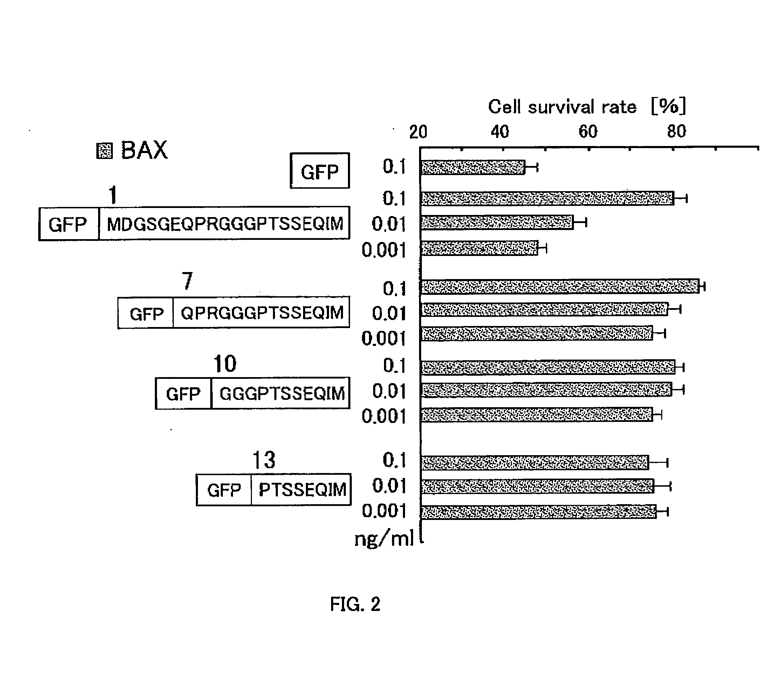

Thus, to vary the amount of the plasmid to be introduced into cells and thereby to examine apoptosis-suppressive activity at each amount of plasmid, each plasmid was transfected into cells in the same manner as described above and the cell survival rate was evaluated by the trypan blue exclusion method. As plasmids to be introduced into cells, pCI-GFP-ART plasmid, pCI-GFP-ART/7-20 plasmid, pCI-GFP-ART/10-20 plasmid, and pCI-GFP-ART/13-20 plasmid were used. The results are shown in FIG. 2. As a result, when 0.01 ng/ml pCI-GFP-ART plasmid was introduced, Bax-induced apoptosis-promoting activity was hardly inhibited. In contrast, when the mutated ART plasmids were introduced, even at a concentration as low as 0.01 ng/ml, the same level of apoptosis-suppressive activity as that seen when a high concentration of pCI-GFP-ART plasmid was introduced was observed.

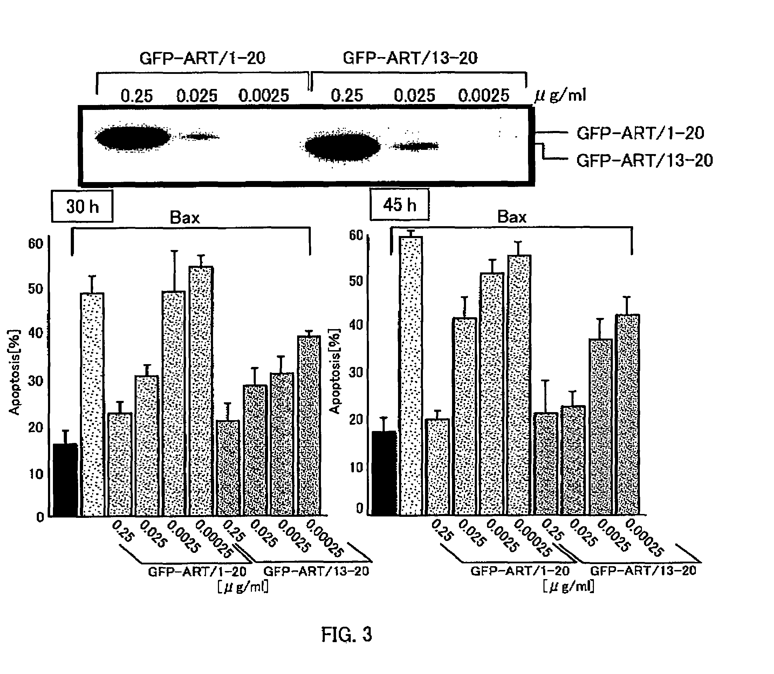

Next, it was confirmed that there was no difference among the plasmids in the level of expression by the plasmids introduced, as follows. HEK 293T cells were plated at 4 x 10⁵ cells per 60 mm Petri dish and incubated overnight. Subsequently, cells were transfected with pCI-GFP-ART plasmid or pCI-GFP-ART/13-20 plasmid and pCI-Bax plasmid by lipofection using opti-MEM (manufactured by GibcoBRL) and Lipofectamine (manufactured by GibcoBRL). After five hours, the medium was changed to DMEM (manufactured by Nissui Pharmaceutical Co., Ltd.) containing 10% FBS (manufactured by Bioserum). After 24 hours, cells were recovered and solubilized in RIPA buffer (50 mM Tris, 125 mM NaCl, 0.5% NP-40, 0.1 g/ml leupeptin, 1 mM PMSF, pH 6.8) to obtain cell lysate, to which half the volume of SDS-sample buffer was added to obtain samples for electrophoresis. SDS-PAGE was performed using these samples and Western blotting was performed with anti-GFP monoclonal antibody (manufactured by Clontech) to examine the expression level of each sample. Further, the survival rate of cells into which plasmids had been introduced was evaluated by the trypan blue exclusion method after 30 and 40 hours. The results are shown in FIG. 3. These results revealed that the expression levels of the wild type ART and mutated ART are almost the same when the same volume of plasmid was introduced, indicating that the expression level of the mutated ART also varies with the amount of plasmid introduced into cells. Consequently, it was found that the mutated ART exhibit a 10- to 100-fold stronger apoptosis-suppressive activity than the wild-type ART.

### EXAMPLE 3

### Preparation of HTA1320

Next, to examine whether the peptide (SEQ ID NO: 2) consisting of the amino acid sequence from position 13 to position 20 of a Bax ART domain is useful for apoptosis-induced diseases, a peptide consisting of the amino acid sequence from position 13 to position 20 of a Bax ART domain was prepared as follows. It should be noted that, in this Example, to enhance cell-permeability, the HIV-Tat sequence was linked to the N-terminus of HTA1320. Further, to facilitate peptide purification, a His sequence was added.

### 3-1 Construction of HTA1320 expression vector

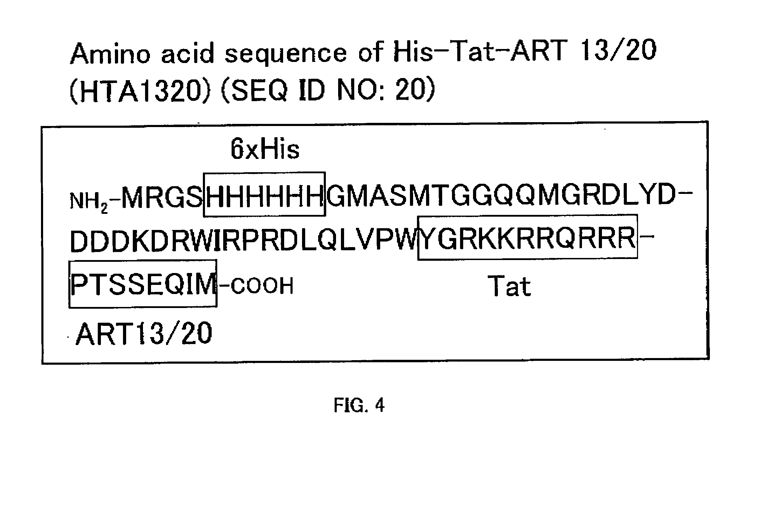

PCR reactions were performed using antisense primer T3 and sense primer TAT-ART-ART/13-20(s) having the HIV-TAT sequence on the 5' end and located downstream of ART using AccuPower PCR PreMix (manufactured by BIONEER) and using pCI-ART (manufactured by Clontech) as template, to obtain the insert, TAT-ARTI320 (SEQ ID NO: 19) (refer to FIG. 4), which was subcloned into pRAET C (manufactured by Invitrogen) to obtain pRAETC-TAT-ART13/20.

TAT-ART/13-20(S):
5'-CGGCGTATAGCTAGGATCCAGCAAGAATTCGTATGGCAGGAAGAAGCGG
AGACAGCGACGCAGACCCACCAGCTCTGAGCAGATC-3' (SEQ ID NO: 21)

T3:
5′-ATTAACCCTCACTAAAGGGAA-3′ (SEQ ID NO: 22)

### 3-2 Purification of HTA1320

After incubating Escherichia coli strain BL21 (DE3) pLysS (manufactured by Invitrogen) into which pRSET C-TAT-ARTI3/20 had been introduced, the expression of His-TAT-ART13/20 (SEQ ID NO: 20) was induced at OD₆₀₀ = 0.6 to 0.7 by adding 1 mM isopropyl-β-D-thiogalactopyranoside (IPTG) (manufactured by Stratagene). The addition of IPTG was followed by 6 hour incubation and then the bacterial cells were recovered. The cells were solubilized with lysis buffer (8 M urea, 100 mM NaCl, 20 mM Hepes, pH 8.0) and the cell lysate was applied to an ni-NTA agarose column (manufactured by QIAGEN). The column was washed with lysis buffer containing 25 mM imidazole, followed by elution with the lysis buffer containing the 250 mM imidazole. The eluted fractions were desalted on a PD-10 column (manufactured by Amersham Biosciences) to obtain pure HTA1320 (SEQ ID NO: 20) (refer to FIG. 4).

### EXAMPLE 4

### Evaluation of apoptosis-suppressive activity of HTA1320

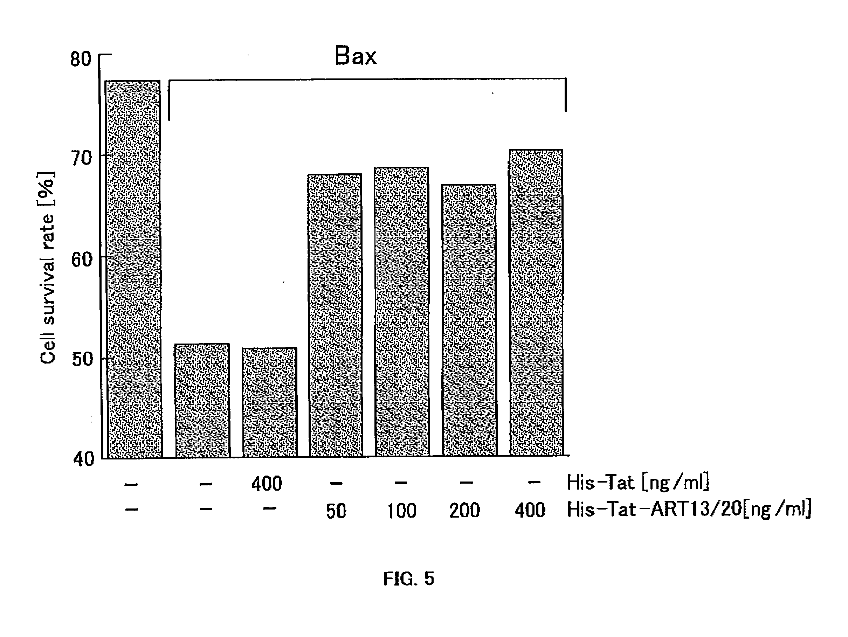

It was examined whether or not HTAI320, prepared in Example 3, inhibits Bax-induced apoptosis-promoting activity. HEK 293T cells were plated at 1 x 10⁵ cells per well on 12-well plastic dishes and incubated overnight. Subsequently, cells were transfected with pCI-Bax by lipofection using opti-MEM (manufactured by GibcoBRL) and Lipofectamine (manufactured by GibcoBRL). After five hours, the medium was changed and HTA1320 were added at various concentrations. Subsequently, the cell survival rate after 40 hours was evaluated by the trypan blue exclusion method. The results are shown in FIG. 5. As a result, HTA1320 inhibited Bax-induced apoptosis-promoting activity. Further, it was found that HTA1320 exhibits, even at low concentrations, apoptosis-suppressive activity almost equivalent to that at high concentrations.

### EXAMPLE 5

### Effect of TAT-ART1320 on Bax-induced apoptosis

Further, it was examined whether the peptide consisting of the amino acid sequence from position 13 to position 20 of ART can suppress apoptosis induced by the overexpression of Bax. It should be noted that, in this example, TAT-ART1320 (SEQ ID NO: 19) synthesized with a peptide synthesizer was used for the following experiments.

### 5-1 Construction of pCI-myc, pCI-myc-Bax, or pCI-myc-Bak plasmid

DNA fragments encoding myc-tag were subcloned into EcoRI-digested and blunted pCI-neo vector to generate pCI-myc vector.

In addition, pGEM-T Easy-Bax (provided from Dr. Siro Shimizu, RIKEN) was treated with EcoRI and then subcloned into the EcoRI site of pCI-myc vector to generate pCI-myc-Bax plasmid.

PCR was performed using forward and reverse primers and using pCMV-bak (provided from Dr. Yoshihide Tsujimoto, Osaka University Medical School) as template. The products were treated with EcoRI, subcloned into the EcoRI site of pCI-myc vector to generate pCI-myc-Bak plasmid.

Forward primer:
5′-CCGTATCGAATTCGATTATGGCTTCGGGGCAAGGCCCAG-3′ (SEQ ID NO: 2)
3)

Reverse primer:
5′-CCGACGCGAATTCACTAGTGATTTCATGATTTGAAGAATC-3′ (SEQ ID: NO)
24)

### 5-2 Experiment

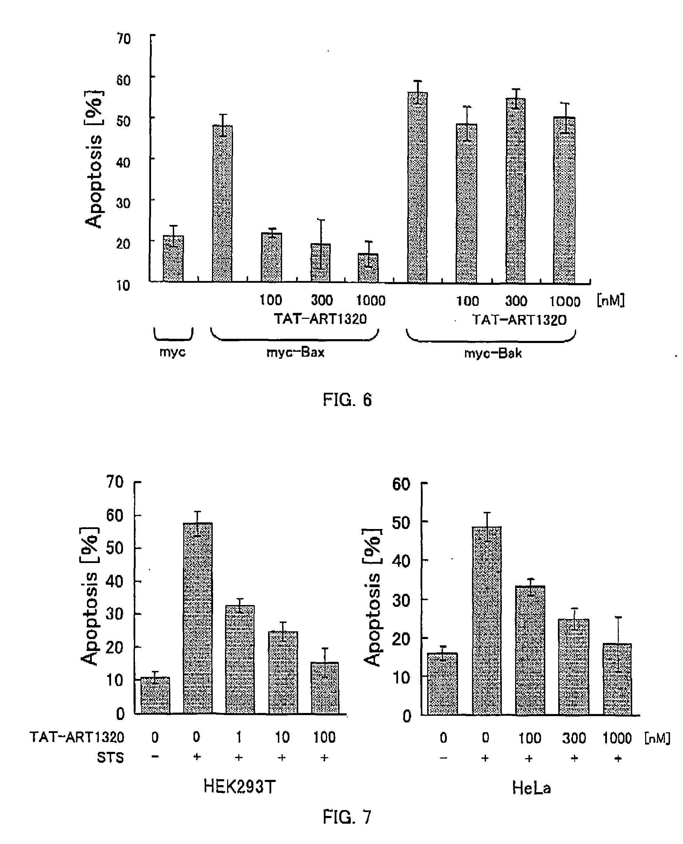

HEK 293T cells (1 x 10⁵ cells) were seeded in 12-well plates and incubated for 24 hours. Subsequently, myc, myc-Bax, or myc-Bak was overexpressed within cells by introducing pCI-myc, pCI-myc-Bax, or pCI-myc-Bak plasmid respectively into HEK 293 T cells. After incubation for 17 hours, TAT-ART1320 was added at a final concentration of 100, 300, or 1000 nM, followed by further incubation for 24 hours. Cells without the addition of TAT-ART1320 were used as controls. Following incubation, cell death was evaluated by the trypan blue exclusion method. The results revealed that, as shown in FIG. 6, TAT-ART1320 inhibits apoptosis induced by the overexpression of Bax but cannot inhibit apoptosis induced by the overexpression of Bak. Thus, it was found that the peptide that consists of the amino acid sequences at positions 13 to 20 can specifically suppress apoptosis induced by the overexpression of Bax.

### EXAMPLE 6

### Suppressive effect of TAT-ARTI320 on STS-induced Bax-dependent apoptosis

It is known that apoptosis induced by staurosporine (STS) is Bax-dependent. Thus, it was examined whether TAT-ART1320 can suppress STS-induced apoptosis.

HEK 293T cells or HeLa cells, at 5 x 10⁴ cells each, were seeded in 12-well plates and incubated for 24 hours. Subsequently, cells were pre-treated by adding TAT-ART1320 at a final concentration of 100, 300, or 1000 nM, followed by further incubation for 24 hours. Then STS was added to HEK 293T cells and HeLa cells at 0.5 µM and 0.2 µM, respectively, followed by incubation for 24 hours. Cells without the addition of TAT-ART1320 or STS, together with cells without the addition of TAT-ART1320, were prepared as controls. Following incubation, cell death was evaluated by the trypan blue exclusion method. The results revealed that, as shown in FIG. 7, TAT-ART1320 suppresses STS-induced apoptosis at concentrations of 1-100 nM and at 100-1000 nM in HEK 293T cells and in HeLa cells, respectively.

### EXAMPLE 7

### Effect of TAT-ART1320 on STS-induced mitochondrial translocation of Bax

It is considered that, in order for Bax to induce apoptosis, it is essential for Bax to localize to mitochondria. Thus, the effect of TAT-ART1320 on STS-induced mitochondrial translocation of Bax was examined.

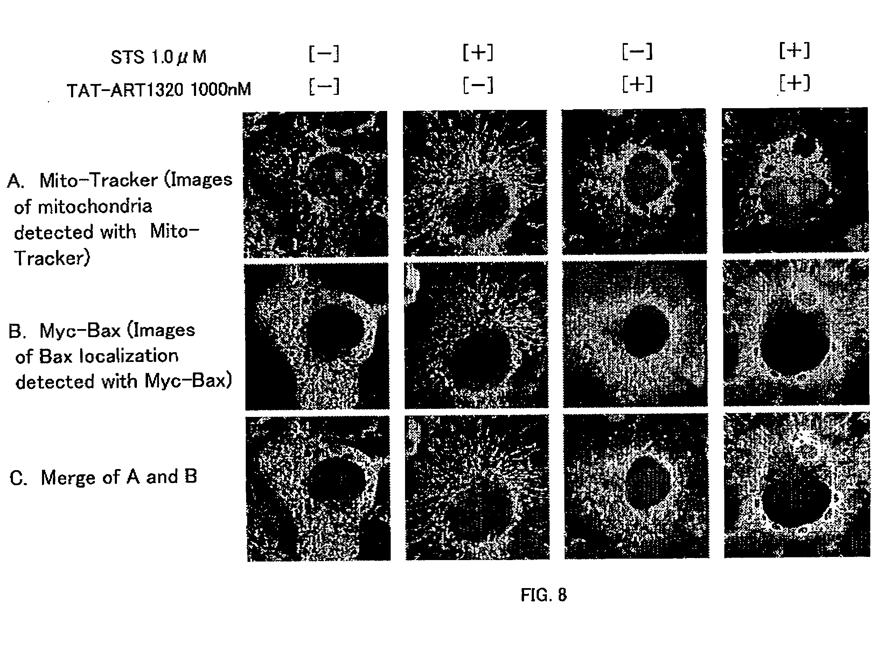

COS7 cells (4 x 10⁴ cells) were seeded in 12-well plates and incubated for 24 hours and then transfected with 1 µg of the pCI-myc-Bax gene. Subsequently, after further incubation for 23 hours, cells were pretreated for 1 hour with 1000 nM TAT-ART1320. Cells without TAT-ART1320 pretreatment were also prepared as controls. Further, 1.0 µl of STS and 100 nM MitoTracker Orange (manufactured by Molecular Probes) were added and, after one hour, cells were fixed with 3% formaldehyde solution. Cells without the addition of STS were also prepared as controls. Subsequently, after treatment for 1 hour with an anti-Myc antibody (manufactured by Santa Cruz: a solution diluted 2500-fold with TBS (20 mM Tris, 137 mM NaCl, pH 7.6), cells were treated for another 1 hour with anti-mouse IgG labeled with Alexa Flour 488 (manufactured by Molecular Probes; a solution diluted 2500-fold with TBS containing 3% bovine serum albumin (BSA)). Then the intracellular localization of Bax was observed with a confocal laser-scanning microscope. The results are shown in FIG. 8.
As indicated in FIG. 8, in cells treated with STS, Bax localized to mitochondra, whereas, in cells treated with TAT-ART1320 and STS, Bax did not localize to mitochondria. This revealed that TAT-ART1320 can suppress STS-induced mitochondral translocation of Bax.

### EXAMPLE 8

### Effect of TAT-ART1320, TAT-ART1420, or TAT-ART1320P13V on STS-induced apoptosis

For the purpose of development of a more active peptide, modified TAT-ART1320 peptides were prepared and their suppressive activities on STS-induced apoptosis were examined. The modified TAT-ART1320 peptides used were TAT-ART1420 (SEQ ID NO: 25) and TAT-ART1320P13V (SEQ ID NO: 26), synthesized with a peptide synthesizer.

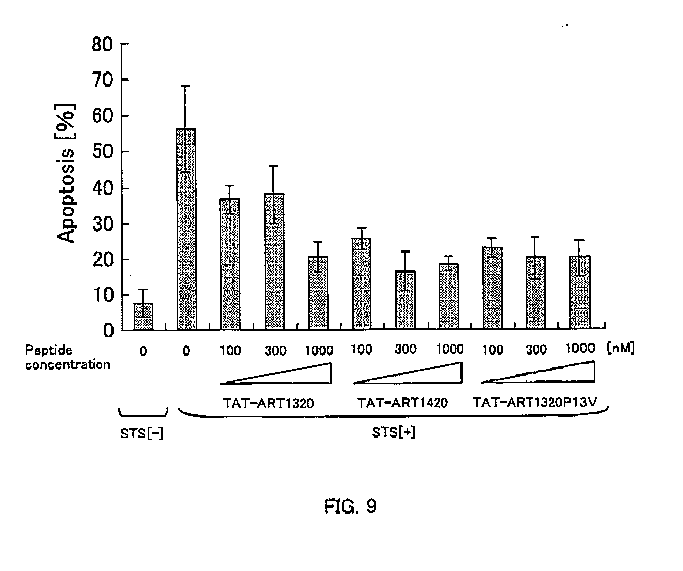

Hela cells (1 x 10⁵ cells) were seeded in 12-well plates and incubated for 24 hours. Subsequently, cells were pre-treated for 1 hour by adding TAT-ART1320 or its modified peptide at a final concentration of 100, 300, or 1000 nM, followed by the addition of 0.2 µM STS and incubation for 24 hours. Cells without the addition of TAT-ART1320 or STS, together with cells without the addition of TAT-ART1320, were prepared as controls. Following incubation, cell death was evaluated by the trypan blue exclusion method. The results are shown in FIG. 9.
As indicated in FIG. 9, TAT-ART1420 and TAT-ART1320P13V suppressed apoptosis to a degree equal to or greater than that of TAT-ART1320. This revealed that, for suppressive activity of apoptosis, the peptide consisting of the amino acid sequences from position 14 to position 20 of an ART sequence is particularly important.

### EXAMPLE 9

### Suppressive effect of TAT-ART1320 in vivo

Next, to confirm the effect of the peptide according to the present invention on apoptosis-induced diseases, such as neurodegenerative diseases, using a model of brain ischemia induced by a 4 min occlusion of the bilateral common carotid arteries in gerbils the influence of TAT-ART1320 (3.30 mg/kg) on the neurological symptom scores after reperfusion and on delayed neuronal death in hippocampus four days after reperfusion.

### 9-1 Test animals

Thirteen-week-old male gerbils (SPF, Japan SLC, Inc.) were kept over a week in a barrier system animal room with controlled temperature (23 ± 2°C), humidity (55 ± 15%), ventilation (at least 5 air changes/hour), and light (12 hour light period, 6:30 a.m. to 6:30 p.m.). The gerbils thus maintained, weighing 60 to 80g, were used as test animals. Food and water were provided at libitum.

### 9-2 Bilateral common carotid artery occlusion and subsequent reperfusion

Under ether anesthesia, test animals underwent a midline skin incision in the neck to expose the bilateral internal carotid arteries (the ether anesthesia was stopped on exposure of the bilateral internal carotid arteries), which were occluded bilaterally with artery forceps, to induce brain ischemia. Reperfusion was initiated by removing the artery forceps 4 min after occlusion and the skin was sutured, immediately followed by administration of TAT-ART1320 or saline. Subsequently, test animals were warmed under an infrared lamp until their activity was recovered.

### 9-3 Administration of TAT-ART1320

TAT-ART1320 was prepared for administration by dissolving 3 or 30 mg of TAT-ART1320 in 10 ml of saline. This solution was administered at a dose of 10 ml/kg BW. Immediately after reperfusion TAT-ART1320 was intravenously injected into the caudal vain of each animal at 3 mg/kg BW or 30 mg/kg BW, followed by another intravenous injection after 5 hours. (The animals (n = 13) receiving TAT-ART1320 at 3 mg/kg BW and animals receiving TAT-ART1320 at 30mg/kg BW were called the 3 mg/kg group and the 30 mg/kg group, respectively.) The next day, intravenous injection was switched to subcutaneous injection and the same doses of TAT-ART1320 were administered twice at intervals of 5 hours, followed by once-daily SC injection. Animals (n = 13: control group) receiving only saline were used as controls.

### 9-4 Symptom observation

Immediately after reperfusion and subsequent administration of TAT-ART1320 or saline, observation of symptoms of the central nervous system (disturbance of consciousness, convulsions, suppression of motor responses, blepharoptosis) was started. The symptoms were observed 30 min, 1 hour, 2 hours, 4 hours, or 6 hours, followed by once-daily observation on subsequent days. Scores were assigned to each of the symptoms according to the degree and duration of their manifestation and a total score was obtained by summing the individual scores.
As a result, the control group (n = 13) undergoing brain ischemia scored 8.2 ± 1.4 for neurological symptoms scores, such as disturbance of consciousness, a convulsive attack, and blepharoptosis. Test animals with TAT-ART1320 administered exhibited a TAT-ART1320-dose-dependent decrease in their scores for neurological symptoms and the 30 mg/kg group exhibited a particularly significant decrease (2.8 ± 0.6, P < 0.05).

### 9-5 Brain sampling

Four days after brain ischemia and subsequent reperfusion, gerbils were anesthetized with ether and the brain was fixed by perfusing transcardially with 10% neutral buffered formalin for 15 min. And then the whole brain was removed. The brain thus removed was firmly postfixed by immersion in 10% of neutral buffered formalin for at least 1 day and used in the following experiments.

### 9-6 Histopathological examination

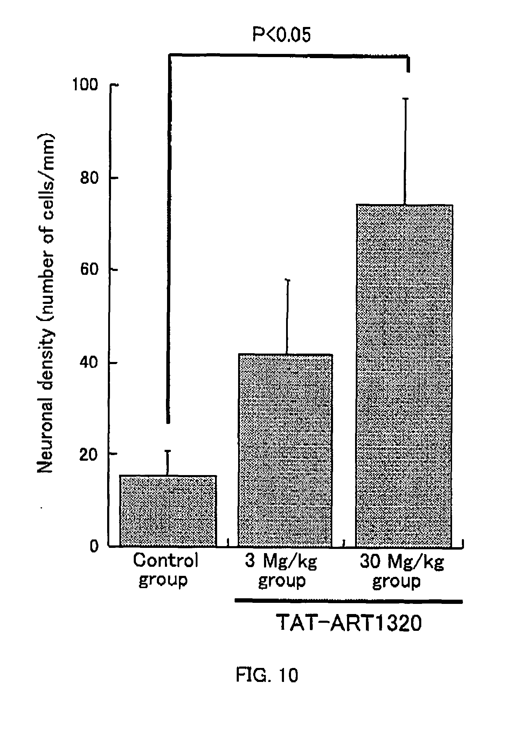

The bilateral hippocampal regions were excised from the fixed brain and 4 µm paraffin sections (about 1.5 mm posterior from the bregma) were prepared from each hippocampus. Kluver-Barrera staining was performed using cresyl violet (Nissl substance staining) and Luxol fast blue (myelin sheath staining). Subsequently, the length of bilateral hippocampal CA1 regions was measured with an image analyzer (MacSCOPE). Further, the number of survived neurons in each section was counted under a microscope. The mean value of the number of survived neurons per unit length present in the bilateral hippocampal CA1 regions was taken as the number of survived neurons in each individual gerbil. As a result, as shown in FIG. 10, the number of survived neurons (SNs) in the 30 mg/kg group (74.5 ± 23.1) was far larger than that in the control group (15.4 ± 5.2), indicating that TAT-ART1320 significantly suppresses neuronal death in the hippocampal CA1 of the brain in a dose-dependent manner. This revealed that TAT-ART1320 has a suppressive effect on neuronal death associated with brain ischemia.

Thus, it was found that the peptide according to the present invention, a nucleotide encoding the peptide, and the like have an effect of improving not only functional disorder but also cell death in brain ischemia model gerbils, and are thus useful for apoptosis-induced diseases (e.g., neurodegenerative disease), and the treatment, prevention, or suppression of progression, of such diseases,

### INDUSTRIAL APPLICABILITY

As described above, according to the present invention, peptides with apoptosis-suppressive activity, polynucleotides, recombinant vectors, cells or viruses, fusion polynucleotides, fusion proteins, or suppressors that suppress the mitochondrial translocation of Bax, all of which are useful for apoptosis-induced diseases and for the treatment, prevention, or suppression of progression, of such diseases, together with suppression of apoptosis-promoting activity.

## Claims

1. A peptide consisting of part of the amino acid sequence of SEQ ID No: 8 and comprising the amino acid sequence shown in SEQ ID No: 1.

2. A peptide consisting of part of the amino acid sequence of SEQ ID No: 8 and comprising the amino acid sequence shown in SEQ ID No: 2.

3. A peptide consisting of an amino acid sequence shown in any one of SEQ ID NOs: 1 to 5.

4. The peptide of any one of claims 1 to 3, comprising an amino acid sequence in which one or a few nucleotides are deleted, substituted, or added, and having apoptosis-suppressive activity.

5. A polynucleotide encoding the peptide of any one of claims 1 to 4, or a polynucleotide comprising a nucleotide sequence complementary to its nucleotide sequence.

6. A recombinant vector comprising the polynucleotide of claim 5.

7. A cell comprising the recombinant vector of claim 6.

8. A virus comprising the recombinant vector of claim 6.

9. A fusion polynucleotide of the polynucleotide of claim 5 and a polynucleotide encoding a tag,
wherein the fusion polynucleotide is constructed such that the peptide can be fused to the tag to be expressed as a polypeptide.

10. A fused protein of the peptide of any one of claims 1 to 4 and a cell-permeable peptide.

11. A pharmaceutical composition for a Bax-dependent apoptosis-induced disease, comprising as an active ingredient at least one substance selected from the group consisting of the peptide of any one of claims 1 to 4, the polynucleotide of claim 5 or 9, the recombinant vector of claim 6, the cell of claim 7, the virus of claim 8, and the fusion protein of claim 10.

12. A pharmaceutical composition, wherein the Bax-dependent apoptosis-induced disease of claim 11 is a disease selected from the group consisting of an ischemic disease, a neurodegenerative disease, diabetes, and an autoimmune disease.

13. A pharmaceutical composition, wherein the Bax-dependent apoptosis-induced disease of claim 11 is a disease selected from the group consisting of myocardial infarction, liver ischemia, brain ischemia, ischemia retinae, Alzheimer's disease, Parkinson's disease, poly glutamine disease, prion disease, amyotrophic lateralsclerosis, acquired immunodeficiency syndrome encephalopathy, multiple sclerosis, and myasthenia gravis.

14. A suppressor that suppresses the mitochondrial translocation of Bax, comprising as an active ingredient at least one substance selected from the group consisting of the peptide of any one of claims 1 to 4, the polynucleotide of claim 5 or 9, the recombinant vector of claim 6, the cell of claim 7, the virus of claim 8, and the fusion protein of claim 10

15. A method for suppressing apoptosis-promoting activity, comprising using at least one substance selected from the group consisting of the peptide of any one of claims 1 to 4, the polynucleotide of claim 5 or 9, the recombinant vector of claim 6, the cell of claim 7, the virus of claim 8, and the fusion protein of claim 10

16. A method for treating, preventing, or suppressing progression of a Bax-dependent apoptosis-induced disease, the method comprising using at least one substance selected from the group consisting of the peptide of any one of claims 1 to 4, the polynucleotide of claim 5 or 9, the recombinant vector of claim 6, the cell of claim 7, the virus of claim 8, and the fusion protein of claim 10.


SEQUENCE LISTING

<110>  KEIO UNIVERSITY

<120>  Peptido with apoptosis-inhibitory activity

<130>  PCT768

<150>  JP 2003/180131
<151>  2003-06-24

<160>  26

<170>  PatentIn version 3.1

<210>  1
<211>  7
<212>  PRT
<213>  Homo sapiens

<220>
<223>  Inventor: Imoto, Masaya
       Invontor: Tabe, Hirokazu
       Inventor: Tashiro, Etsu
       Inventor: OHMORI, Yusuke

<400>  1

Thr Ser Ser Glu Gln Ile Met
1               5


<210>  2
<211>  8
<212>  PRT
<213>  Homo sapiens

<400>  2

Pro Thr Ser Ser Glu Gln Ile Met
1               5


<210>  3
<211>  11
<212>  PRT
<213>  Homo sapiens

<400>  3

Gly Gly Gly Pro Thr Ser Ser Glu Gln Ile Met
1               5                   10


<210>  4
<211>  14
<212>  PRT
<213>  Homo sapiens

<400>  4

Gln Pro Arg Gly Gly Gly Pro Thr Ser Ser Glu Gln Ile Met
1               5                   10


<210>  5
<211>  17
<212>  PRT
<213>  Homo sapiens

<400>  5

Ser Gly Glu Gln Pro Arg Gly Gly Gly Pro Thr Ser Ser Glu Gln Ile
1               5                   10                  15

Met

<210> 6
<211> 8
<212> PRT
<213> Artificial Sequence

<220>
<223> Mutant peptide

<400> 6

Pro Thr Ala Ala Glu Gln Ile Met
1               5

<210> 7
<211> 8
<212> PRT
<213> Artificial Sequence

<220>
<223> ART1320P13V

<400> 7

Val Thr Ser Ser Glu Gln Ile Met
1               5

<210> 8
<211> 20
<212> PRT
<213> Homo sapiens

<400> 8

Met Asp Gly Ser Gly Glu Gln Pro Arg Gly Gly Gly Pro Thr Ser Ser
1               5                   10                  15

Glu Gln Ile Met
            20

<210> 9
<211> 60
<212> DNA
<213> Homo sapiens

<400> 9
atggacgggt ccggggagca gcccagaggc gggggcccca ccagctctga gcagatcatg      60

<210> 10
<211> 31
<212> DNA
<213> Artificial Sequence

<220>
<223> GFP-ART(S)

<400> 10
cggtatcaag ttatagctag cgctaccggt c                                     31

<210> 11
<211> 89
<212> DNA
<213> Artificial Sequence

<220>
<223> GFP-ART(AS)

<400> 11
ccgctagcta catgatctgc tcagagctgg tgggcccccc gcctctgggc tgctccccgg 60

accogtccat cttgtacagc tcgtccatg 89


<210> 12
<211> 40
<212> DNA
<213> Artificial Sequence

<220>
<223> GFP(S)

<400> 12
ccgtagcgat aaggaattca tggtgagcaa gggcgaggag 40


<210> 13
<211> 43
<212> DNA
<213> Artificial Sequence

<220>
<223> GFP-ART/1-9(AS)

<400> 13
cggctatcgg ttacatgaat ccatggtgag caagggcgag gag 43


<210> 14
<211> 46
<212> DNA
<213> Artificial Sequence

<220>
<223> GFP-ART/1-16(AS)

<400> 14
cggtattact aatctcgcaa ttcctaagag ctggtgggcc ccccgc 46


<210> 15
<211> 99
<212> DNA
<213> Artificial Sequence

<220>
<223> GFP-ART/4-20(AS)

<400> 15
cggagttaga tacatatcaa gaattcctac atgatctgct cagagctggt gggccccccg 60

cctctgggct gctccccgga cttgtacagc tcgtccatg 99


<210> 16
<211> 91
<212> DNA
<213> Artificial Sequence

<220>
<223> GFP-ART/7-20(AS)

<400> 16
cggacgtagc gagatatagt agaattccta catgatctgc tcagagctgg tgggcccccc 60

gcctctgggc tgcttgtaca gctcgtccat g 91


<210> 17
<211> 77
<212> DNA
<213> Artificial Sequence

<220>

<223>  GFP-ART/10-20(AS)

<400>  17
ccgattaccg cttagagaat tcctacatga tctgctcaga gctggtgggc cccccgccct      60

tgtacagctc gtccatg                                                    77


<210>  18
<211>  70
<212>  DNA
<213>  Artificial Sequence

<220>
<223>  GFP-ART/13-20(AS)

<400>  18
ccgcgtacga tggatgtcga attcctacat gatctgctca gagctggtgg gcttgtacag      60

ctcgtccatg                                                            70


<210>  19
<211>  19
<212>  PRT
<213>  Artificial Sequence

<220>
<223>  TAT-ART1320

<400>  19

Tyr Gly Arg Lys Lys Arg Arg Gln Arg Arg Arg Pro Thr Ser Ser Glu
1               5                   10                  15


Gln Ile Met



<210>  20
<211>  64
<212>  PRT
<213>  Artificial Sequence

<220>
<223>  His-Tat-ART13/20(HTA1320)

<400>  20

Met Arg Gly Ser His His His His His His Gly Met Ala Ser Met Thr
1               5                   10                  15


Gly Gly Gln Gln Met Gly Arg Asp Leu Tyr Asp Asp Asp Asp Lys Asp
            20                  25                  30


Arg Trp Ile Arg Pro Arg Asp Leu Gln Leu Val Pro Trp Tyr Gly Arg
        35                  40                  45


Lys Lys Arg Arg Gln Arg Arg Arg Pro Thr Ser Ser Glu Gln Ile Met
    50                  55                  60


<210>  21
<211>  85
<212>  DNA
<213>  Artificial Sequence

<220>
<223>  TAT-ART/13-20(S)

<400>  21
cggcgtatag ctaggatcca gcaagaattc gtatggcagg aagaagcgga gacagcgacg      60

cagacccacc agctctgagc agatc                                                85

<210>  22
<211>  21
<212>  DNA
<213>  Artificial Sequence

<220>
<223>  T3

<400>  22
attaaccctc actaaaggga a                                                    21

<210>  23
<211>  39
<212>  DNA
<213>  Artificial Sequence

<220>
<223>  Forward primer

<400>  23
ccgtatcgaa ttcgattatg gcttcggggc aaggcccag                                 39

<210>  24
<211>  40
<212>  DNA
<213>  Artificial Sequence

<220>
<223>  Reverse primer

<400>  24
ccgacgcgaa ttcactagtg atttcatgat ttgaagaatc                                40

<210>  25
<211>  18
<212>  PRT
<213>  Artificial Sequence

<220>
<223>  TAT-ART1420

<400>  25

Tyr Gly Arg Lys Lys Arg Arg Gln Arg Arg Arg Thr Ser Ser Glu Gln
1               5                   10                  15

Ile Met

<210>  26
<211>  19
<212>  PRT
<213>  Artificial Sequence

<220>
<223>  TAT-ART1320P13V

<400>  26

Tyr Gly Arg Lys Lys Arg Arg Gln Arg Arg Arg Val Thr Ser Ser Glu
1               5                   10                  15

Gln Ile Met